# EUROPEAN PATENT SPECIFICATION

(11) **EP 4 466 035 B1**
(45) Date of publication and mention of the grant of the patent: **05.03.2025**
(21) Application number: 23718370.2
(22) Date of filing: 17.04.2023
(51) Int. Cl.: A61L 31/14, B22F 3/24, A61L 27/04, A61L 31/02, B22F 3/20, C22C 1/10, C22C 18/00, C22C 32/00, C22C 1/04, B22F 1/052

(54) **A BIOCOMPATIBLE AND BIOABSORBABLE METAL MATRIX COMPOSITE FOR MANUFACTURING BIOMEDICAL ELEMENTS AND METHOD OF MANUFACTURE OF THE SAME**
BIOKOMPATIBLES UND BIOABSORBIERBARES VERBUNDMATERIAL ZUR VOLLSTÄNDIGEN ABSORPTION IN VIVO BEI KONTAKT MIT MENSCHLICHEM ODER TIERISCHEM GEWEBE UND VERFAHREN ZUR HERSTELLUNG DIESES VERBUNDMATERIALS
MATÉRIAU COMPOSITE BIOCOMPATIBLE ET BIOABSORBABLE POUR ABSORPTION TOTALE IN VIVO EN CONTACT AVEC UN TISSU HUMAIN OU ANIMAL ET PROCÉDÉ DE FABRICATION DUDIT MATÉRIAU COMPOSITE

(30) Priority: 19.08.2022 EP 22191338
(43) Date of publication of application: 27.11.2024
(73) Proprietor: Centrum pre vyuzitie pokrocilych materialov Slovenskej akademie vied, verejna vyskumna institucia, 845 11 Bratislava - mestska cast Karlova Ves (SK); Ustav materialov a mechaniky strojov Slovenskej akademie vied, verejna vyskumna institucia, 845 13 Bratislava - mestska cast Karlova Ves (SK)
(72) Inventor: BALOG, Martin, 841 07 Bratislava (SK); KRIZIK, Peter, 902 03 Pezinok (SK)
(74) Representative: Majlingová, Zuzana
(86) International application number: PCT/SK2023/050007
(87) International publication number: WO 2024/039303

(56) References cited:
- WO-A1-2021/243398
- CN-A- 114 411 014
- US-A1- 2021 353 835
- JARZEBSKA A ET AL: "A new approach to plastic deformation of biodegradable zinc alloy with magnesium and its effect on microstructure and mechanical properties", MATERIALS LETTERS, vol. 211, 15 January 2018 (2018-01-15), pages 58 - 61, XP085274102, ISSN: 0167-577X, DOI: 10.1016/J.MATLET.2017.09.090
- MOSTAED EHSAN ET AL: "Zinc-based alloys for degradable vascular stent applications", ACTA BIOMATERIALIA, ELSEVIER, AMSTERDAM, NL, vol. 71, 10 March 2018 (2018-03-10), pages 1 - 23, XP085377867, ISSN: 1742-7061, DOI: 10.1016/J.ACTBIO.2018.03.005
- MCCARTHY W. H. ET AL: "Compression Strength of Zinc/Zinc Oxide Composites at Temperatures above the Melting Point of Zinc", vol. 4, no. 1, 18 January 1970 (1970-01-18), pages 74 - 77, XP093016408, ISSN: 0026-0681, Retrieved from the Internet <URL:http://dx.doi.org/10.1179/030634570790444086> DOI: 10.1179/030634570790444086

## Description

### Technical filed

The invention relates to bioabsorable zinc (Zn)-based composite materials for biomedical applications.

### Background art

Coronary artery disease (CAD) is the foremost cause of death worldwide, creating a major economic burden on patients' quality of life and public health systems [1]. Common way to treat CAD is stenting by traditional inert metals (stainless steel or cobalt-chromium alloys). Inert endovascular stents (ES) stay in the human body for many years and causes long-term health risks such as impaired coronary vasomotion, development of early neoatherosclerosis, late-stage thrombosis, chronic inflammation, etc. [2]. Furthermore, the surgical implementation of internal fixation metallic implants (wires, plates, screws, staples) is commonly applied in orthopedics for the purpose of repairing a bone. After the time, which is necessary to support a recovering tissue, orthopedic internal fixators (OIF) become redundant; its long-term retention becomes problematic e.g., the refracture rate increases and has to be removed. However, a secondary operation is most stressful to a patient, and brings economical burdens.

Owing to their unique combination of properties metals represent traditionally an appealing option for bioabsorbable implant applications in particular low profile OIF and ES with a thin strut section. Among other bioabsorbable metals, namely magnesium (Mg) and iron (Fe), Zn has attracted considerable increase in scientific interest recently as it addresses some of the major issues related with using of Mg and Fe biometals [3].

Zn, which is considered as a nutrition essential trace element in the human body, plays structural, regulatory, and catalytic roles in the human cell [4]. Zn resorbs at the rate, which falls between: i) the difficult to control high corrosion rate of Mg and ii) the degradation rate of Fe, which is too slow [3]. The intermediate corrosion rate of Zn provides an engineering advantage to design a low-profile ES (with a requested penetration rate (<20 µm·y⁻¹) and OIF (0.5 µm·y⁻¹), which retain mech. integrity and full absorption for required times of 3-6 months and 1-2 years, respectively [3, 5]. Owing to a low melting point (Tₘ) and low reactivity, Zn can be processed easily and simply by conventional techniques of casting and wrought processing.

However, bioabsorbable Zn materials are not without shortcomings. Most importantly pure Zn shows rather low mechanical performance compared to Fe and Mg, which may jeopardize mechanical integrity namely of low-profile ES [3].

Alloying of Zn with elements, which are biocompatible or essential to human function, is the most logical solution applied by many, which increases the poor strength of pure Zn [3]. Another feasible approach is cold/warm working of coarse-grained Zn structures by e.g, drawing, direct hydroextrusion (HE) or conventional extrusion (CE) operations at high strains and low strain rates resulting in refined, equiaxed (sub)grain structure, which assures a significant strength increase accompanied with reasonably high ductility (ε) [3].

But for all of these developed Zn alloys some major problems remain unresolved. It was shown that refined ZnMg structures are prone to post-deformation instability of mechanical properties, so-called "annealing strengthening", which limits application potential of Zn biometals [6]. Herein, the significant strengths increase and severe drop of ε were observed after post-deformation lagering (even short term), due to the natural age hardening i.e., overaging related to grain structure regeneration and Zn₁₁Mg₂ precipitation processes. As a result of a low recrystallization temperature of Zn (~ 10 °C), highly strained non-equilibrium ultrafine-grained (UFG) Zn structures undergo changes related to grain structure regeneration at the temperature of a human body (THB). Non-equilibrium severely deformed UFG Zn alloy structures boost the dynamic precipitation and are prone to precipitation softening [9]. Expectedly, near and submicrometre Zn grain structures tend to creep profoundly at the temperature of a human body, yet the creep results on bioabsorbable Zn alloys are scarce [7].

Besides, an increased induced strain gave a rise of structural inhomogeneity i.e., an existence of shear bands with pronounced tendency to dynamic recrystallization (DRX), and flow localization, which in turn affected workability [8]. Furthermore, it was reported Zn biometals being prone to the strain softening, while it may have a negative impact on uniformity of expansion and recoil of ES [6]. The mechanical properties of Zn are strongly strain rate (ε^{.}) sensitive and steeply deteriorate with an increase of temperature, what bears important implications on the processability and applicability [6].

US 2021/0353835 A1 discloses a biomedical device that includes a Zn-based matrix and nanostructures dispersed in the matrix and a method of its production. It discloses a broad family of bioresorbable Zn based metal matrix composites (MMC) with fine grained Zn grain structure (1-1000 µm) stiffened, stabilized and strengthened by nanocomponent (<1 µm) of a broad range of ceramic particles (carbides, oxides, etc.) with the content >2.5vol.% fabricated by combination of solid state and molten routes mostly for biomedical applications.

However, there are several disadvantages of the disclosed device and method. Firstly, there is a limitation in a minimal grain size, which can be attained by the process. Major improvement in terms of strength, fatigue and creep endurance takes place for UFG structures i.e., for the grain size <1 µm. Furthermore, ceramic reinforcement increases Young's modulus (E) of the composite, whereas E shall rather decrease in order to avoid the problems related to a stress shielding effect in OIF. The ex-situ fabrication process poses a high risk of reinforcement clustering and minimal reinforcement's size to be accommodated successfully, with an expected negative effect on performance and toxicity. Other risk related to process carried out at elevated temperatures is formation of unwanted products due to reaction at matrix-reinforcement interface. Other limitation is that some enclosed reinforcement systems are not biocompatible and may induce a toxic effect by various means upon Zn matrix resorption.

The aim of this invention is to provide a Zn-based bioabsorbable MMC capable of dissolving fully and safely in the body at a moderate absorption rate, with improved mechanical properties due to better stability of composite grains.

### Summary of the Invention

It has been surprisingly found out that the mechanical properties, in particular a stability, of the bioabsorable Zn-based MMC are considerably improved if the MMC is prepared in by the method where:
a powder,
- having a mean particle size in the range from 50 to 10000 nm, where powder particles consist of a core and an outer layer, and where the core is selected from the group consisting of: Zn, ZnMg alloy, ZnCa alloy; and the outer layer is selected from the group consisting of: ZnO, MgO, ZnHPO₄; and where the outer layer constitutes 0.1 to 10 vol. % of the powder particles;
is compacted by cold isostatic pressing at a pressure from 20 to 400 MPa and subsequently extruded at a temperature below melting point of the powder at a reduction ratio 5:1 to 100:1, wherein the mean particle size of the powder and the reduction ratio are such as to obtain an ultra-fine grained matrix structure of the processed core material; and wherein fragments of the outer layer are evenly dispersed within the structure.

During the extrusion, the outer layer is fragmented and introduced *in situ* into the refined matrix structure in the form of dispersoids. Dispersoids are of discrete irregular platelets-like form, as they originate from outer layers existing/formed on the surface of Zn or Zn-alloy cores of powder particles.

It was discovered that nanoscale platelet-like fragments of the outer layer (dispersoids), distributed by the above-mentioned process within the UFG Zn structure have a significant stabilizing effect on the UFG Zn based matrix structure - they prevent further growth of the grains of the matrix structure. It has an advantage that the matrix structure does not coarsen during production, or subsequent storage, or during its service in the human body. By preserving the UFG structure of the composite, the long-term mechanical properties of the material are maintained.

According to a preferred embodiment the powder is HE. The extrusion is carried out at the temperature below melting point of the powder, preferably in the range from 4 °C up to melting point of the powder, more preferably in the range from 4 °C to 100 °C, the most preferably at a room temperature. The reduction ratio of the extrusion is 5:1 to 100:1, preferably 10:1 to 20:1, the most preferably 12:1.

Firstly, the powder has to have a mean particle size in the range from 50 to 10000 nm, preferably from 500 to 10000 nm, more preferably from 1000 to 7000 nm, the most preferably around 3000 nm. It is important that a fine powder is used. Such powder, consolidated by the method according to the present invention, at the appropriate reduction ratio, results in UFG (grain size <1 µm, preferably from 50 to 950 nm) matrix structure of the final composite, which determines its positive properties, especially the mechanical properties, but also the corrosion and biological properties of the final MMC.

Secondly, the MMC has to comprise a sufficient amount of the material of the outer layer (0.1 to 10 vol. % of the composite/powder particles), preferably 0.1 to 8 vol. %, or 1 to 7 vol. %, the most preferably 3 to 5 vol. %. of the powder particles. This ensures that in situ created dispersoids are of suitable size (approximately 1 to 500 nm, preferably 1 to 200 nm) and shape (discrete irregular platelets-like). The amount of outer layer material below 0.1 vol. % results in insufficient number of dispersoids that translates into deterioration of the stabilizing effect. On the other hand, if the content of the outer layer exceeds 10 vol. %, the outer layer would be overly thick and the resulting dispersoids too large. Dispersoids larger than 500 nm may act as stress concentrators, promoting formation and propagation of cracks. With large dispersoids, the homogeneity of their distribution also decreases, which also negatively affects the mechanical, corrosion, and biological properties of the material.

Apart from a suitable size and shape, the dispersoids have to be evenly distributed in a specific way within the matrix. The shape and distribution of the dispersoids are very specific and we believe that it significantly contributes to the stability of the composite material. The method according to the present invention ensures that dispersoids of the appropriate size and shape are formed in the appropriate number, and are specifically distributed within the UFG Zn matrix structure.

According to the preferred embodiment, the powder can be cold compacted by a cold isostatic pressing at a pressure from 100 to 300 MPa.

According to the preferred embodiment after the extrusion step, the bioabsorbable MMC is machined or formed to a shape and dimensions of an implant.

The material of the core can be either Zn, ZnMg alloy or ZnCa alloy. The amount of Mg or Ca in the Zn alloy is up to 5 wt.%, preferably 0.5 to 2 wt.%. The material of the outer layer can be ZnO, MgO or ZnHPO₄. The outer layer can be technologically created. In case of the ZnO outer layer, it can be also naturally occurring.

Such composite material is suitable for use in surgery as a biomedical implant such as ES or OIF. Further possible applications of the bioabsorbable MMC according the subject invention are e.g., different purpose stents, bioresorbable dental membranes, functional biodegradable electronics and sensors.

Further aspect of this invention is a method of manufacturing of the bioabsorable Zn-based MMC as disclosed above.

The advantage of the method according to the invention is that it, in an elegant and simple manner, introduces *in situ* at low processing temperature rather small amount of nanometric second phase (dispersoids), which is very fine and dispersed evenly in principle within the matrix. On the top of that, it uses widely available powder component. That all results in low production costs. Moreover, the second phase is biocompatible and bioresorbable in principle, while it improves osseointegration and antibacterial properties at the same time. Refined composite grain structure, fine nature of *in-situ* introduced second phase, its relatively low content and homogeneity across the volume of composite yields superior mechanical performance, incl. fatigue and creep strengths and life, stability of the microstructure pinned by the second phase, hence assures the microstructural stability upon processing, storage and service and its performance, homogenous corrosion at desired rates, non-toxicity, etc.

To summarize, a primary role of disperoids is stabilization of a refined Zn/Zn-alloy grain structure during processing, post-processing, storage, and service of the MMC. In this manner a post-deformation stability of the attractive mechanical properties of MMC against grain coarsening and age-hardening, which may take place during storing and service, is maintained. Dispersoids located in the matrix suppress a grain boundary rotation and sliding creep deformation mechanisms, which in turn improves creep and fatigue performance. Furthermore, a small fraction of dispersoids, which are homogenously dispersed in Zn matrix, pose no major deteriorating effect as stress concentrators and crack initiators on obtained mechanical properties.

As far as it concerns a biological effect of dispersoids, the materials used are nontoxic, soluble and biocompatible. Furthermore, ZnO also provides antibacterial properties, enhances the stem cell proliferation capacity, and provides anticancer properties.

Furthermore, various chemical, physical and mechanical methods can be used to replace or modify native passivating ZnO films present on the surface of as-received (as-atomised) fine Zn and Zn alloy powders. Though, a surface modification of Zn and Zn alloy powder doesn't alter the above discussed concept of in-situ stabilization of refined Zn grain structure by ZnO nano dispersoids, which remain virtually the same.

### Brief description of the drawings

Fig. 1a and 1b - represent Electron Backscatter Diffraction (EBSD) data analysis of HE Zn+ZnO for the transversal direction: IPF map shown in [001] crystal direction.
Fig. 1c - represents a grain size distribution chart with the insert of the corresponding pole figures.
Fig. 2a and 2b - represent EBSD data analysis of HE Zn+ZnO for the longitudinal direction: IPF map shown in [001] crystal direction. The arrow indicates the extrusion direction.
Fig. 3a - represents ADF STEM micrograph of HE Zn+ZnO in transversal direction.
Fig. 3b - represents the corresponding combined EDS map of Zn and O elements of HE Zn+ZnO shown in transversal direction.
Fig. 4 - represents ADF STEM micrograph of ZnO dispersoid and the corresponding combined EDS map of Zn and O elements in HE Zn+ZnO.
Fig. 5 - represents HAADF micrograph of ZnO dispersoid in HE Zn+ZnO with corresponding FFT. The marked area revealing the ZnO dispersoid [100] zone axis embedded within Zn matrix [210] zone axis.
Fig. 6 - displays the representative tensile stress-strain curves of Zn+ZnO in as-extruded state fabricated by hydroextrusion (HE Zn+ZnO) and after annealing at 100 °C for 24 h (HE+A Zn+ZnO). For comparison data for the cast Zn, and HE and HE+A Zn is shown too.
Fig. 7 - displays the representative compressive stress-strain curves of HE Zn+ZnO obtained in the longitudinal and transversal direction in respect to the extrusion axes.

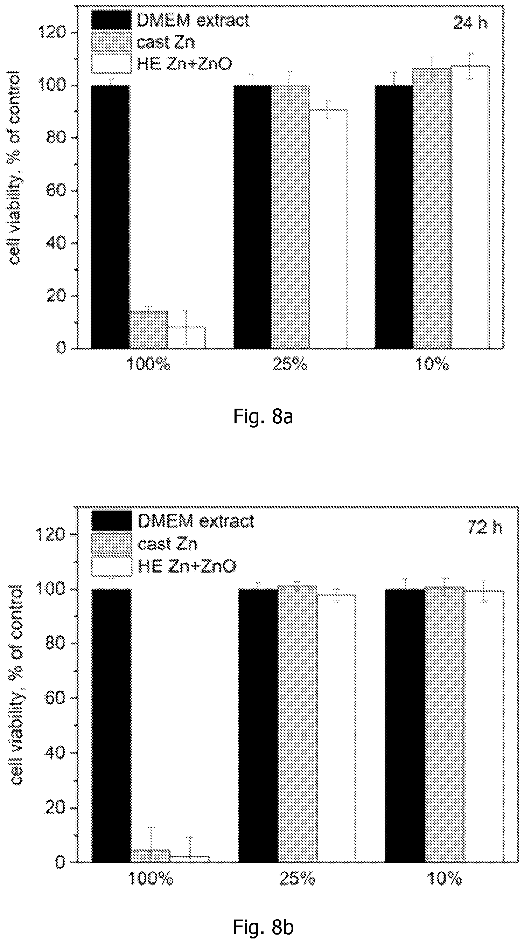

Fig. 8a and 8b - display cell viability of L929 incubated with the undiluted (100%) and diluted (25 and 10%) extracts of the investigated HE Zn+ZnO and cast Zn samples for (a) 24 h and (b) 72 h evaluated by an MTT assay.

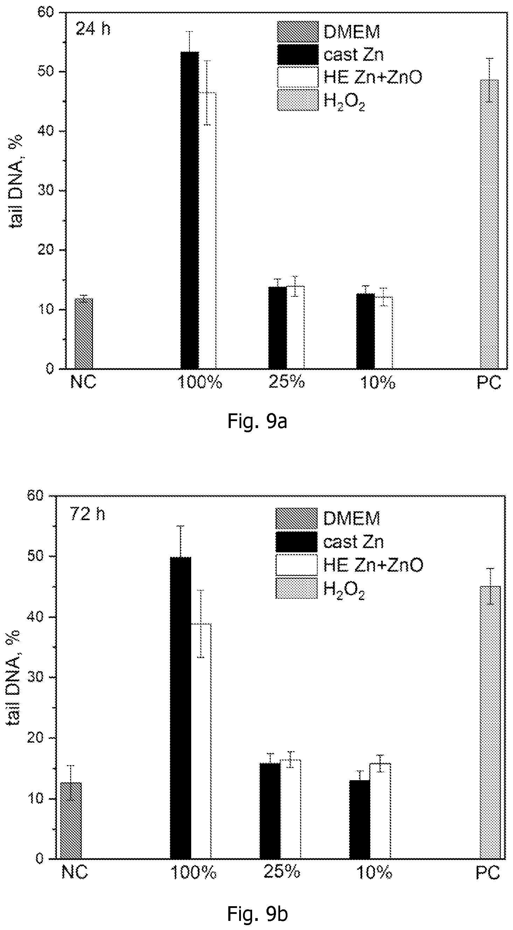

Fig. 9a and 9b - display genotoxic effect of the L929 cells incubated with the concentrated (100%) and diluted (25% and 10%) extracts of the investigated HE Zn+ZnO and cast Zn reference exposed for (a) 24 and (b) 72 h evaluated by comet assay. Negative control (NC): DMEM; positive control (PC): H₂O₂.
Fig. 10a and 10b - display ROS generation potential of the L929 cells incubated with the 25% and 10% extracts of the HE Zn+ZnO and cast Zn samples for (a) 24 and (b) 72 h exposures. Negative control (NC): DMEM; positive control (PC): Menadione.
Fig. 11 - display antibacterial activity of the diluted (25% and 10%) DMEM extracts of the HE Zn+ZnO and cast Zn specimens after 24 h incubation of Staphylococcus aureus in suspension with subsequent colony growth on tryptone soya agar plates. Bacterial colonies were counted and are expressed as the specific (per mL) colony forming units (CFU). Negative control (NC): DMEM.
Fig. 12 - display SEM micrograph of HE+A Zn in the coarse-grained area for the transversal direction.

### Detailed description

### Example 1

Gas atomized 4 N Zn powder (d₁₀, d₅₀ and d₉₀ powder particle size values of 1.35, 3.21 and 7.45 µm) was used. The Zn powder having naturally formed ZnO passivating layer on the surface of individual powder particles. The Zn powder was pre-compacted by cold isostatic pressing at a pressure of 200 MPa and consolidated by HE, which was carried out at room temperature into rod bars with a diameter of 10 mm at a reduction ratio of ~12:1 and a ram speed of 15 mm.s⁻¹.

A part of the profile was further annealed at 100 °C for 24 h in argon. The annealing at 100 °C for 24 h may be considered as an accelerated thermal treatment, which simulates long term service of biomedical device at THB.

### Example 2

Gas atomized 4 N Zn powder (d₁₀, d₅₀ and d₉₀ powder particle size values of 1.35, 3.21 and 7.45 µm) was used. The Zn powder having naturally formed ZnO passivating layer on the surface of individual powder particles. The Zn powder was pre-compacted by cold isostatic pressing at a pressure of 200 MPa and consolidated by conventional extrusion, which was carried out at room temperature into rod bars with a diameter of 10 mm at a reduction ratio of ~12:1 and a ram speed of 15 mm.s⁻¹.

### Comparative Example 1

The cast 4N Zn material was used.

### Comparative Example 2

The cast Zn of comparative Example 1 was pre-compacted by cold isostatic pressing at a pressure of 200 MPa and consolidated by hydro-extrusion, which was carried out at room temperature into rod bars with a diameter of 10 mm at a reduction ratio of ~12:1 and a ram speed of 15 mm.s⁻¹.

### Comparative Example 3

A part of the profile extruded in the comparative example 2 was further annealed at 100 °C for 24 h in argon.

**Table 1 - The designation of Zn+ZnO and Zn samples.**

| **feedstock material** | **condition** | **designation** |
|---|---|---|
| **Zn powder** | hydroextruded | HE Zn+ZnO |
| | hydroextruded + annealed | HE+A Zn+ZnO |
| | conventionally extruded | CE Zn+ZnO |
| **Zn ingot** | cast | cast Zn |
| | cast + hydroextruded | HE Zn |
| | cast + hydroextruded + annealed | HE+A Zn |

### Microstructural analysis

All samples were subjected to microstructural analysis.

Density was determined by Archimedes' principle. The oxygen (O) content was determined by hot gas fusion analysis. The microstructure was examined using a scanning electron microscope. The structure was studied by X-ray diffraction (XRD).

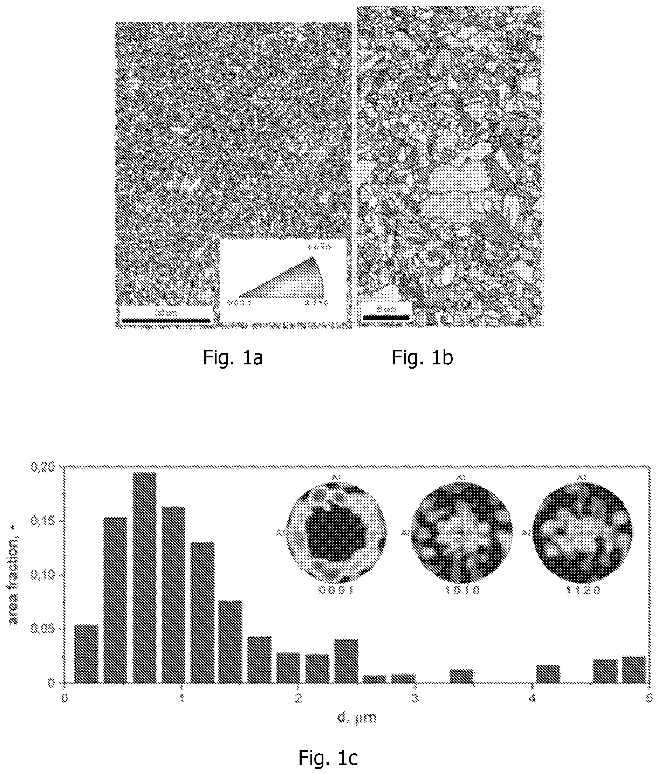

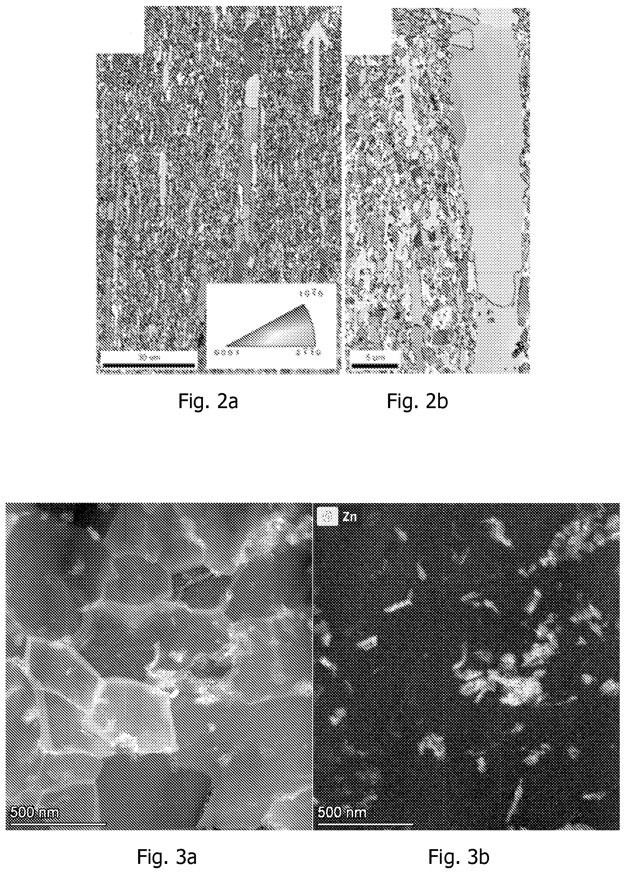

Hydro-extrusion process resulted in almost full densification of Zn powder into a sound material. A measured density of HE Zn+ZnO was 7.014 g.cm⁻³, which represented 98.7% of the theoretical density of Zn composite with the content of ZnO being 2.13 vol.%. The ZnO content was calculated from measured O (0.33±0.03 wt.%), whereas all O was attributed to the presence of ZnO component. **Figs. 1a****, b** and **2a, b** show the grain orientation EBSD IPF maps of HE Zn+ZnO collected in the transversal and longitudinal directions, respectively. Hydrostatic condition and shearing introduced during hydro-extrusion resulted in a pronounced plastic deformation of Zn powder and refined Zn grain structure eventually. The microstructure in the transversal direction consisted of equiaxed Zn grains with an average grain size of 0.75±0.37 µm and rather broad size distribution (**Figs. 1a, b, c**. As determined by the grain orientation spread (GOS) parameter in most of the grains were either fully recrystallized with well-developed high angle grain boundaries (HAGB) and no obvious internal substructure or showed a little increased deformation. The low GOS parameter indicated a dynamic recovery, which took place during HE. In the longitudinal direction Zn grain structure was characterized by a mixture of equiaxed grains and those elongated along the extrusion direction (**Fig. 2a****, b**). The equiaxed grains tended to have a finer size. Conversely, elongated grains were mostly larger, had fragmented structure and were often part of the bands. Again, the GOS map proved that equiaxed fine grains were more uniform, while elongated coarser grains had larger GOS parameter mostly. On one hand, the existence of the bands might be attributed to shearing induced during HE, as often observed for highly deformed Zn-based materials. On the other hand, large elongated grains might stem from coarser Zn powder particles with an average size of ~20 µm, as seen in the bimodal PSD curve of as-atomized Zn powder. **Figs. 1a, 1b, 1c** and **2a, 2b** confirmed that HE Zn+ZnO was significantly textured with the basal planes being parallel with the extrusion direction, which is common for the extruded Zn. Although, the basal planes were inclined from the L direction to the T direction by a relatively large angle, with the maximum intensity at approximately 20°, which was attributed to the activity of the 2^{nd} order pyramidal slip.

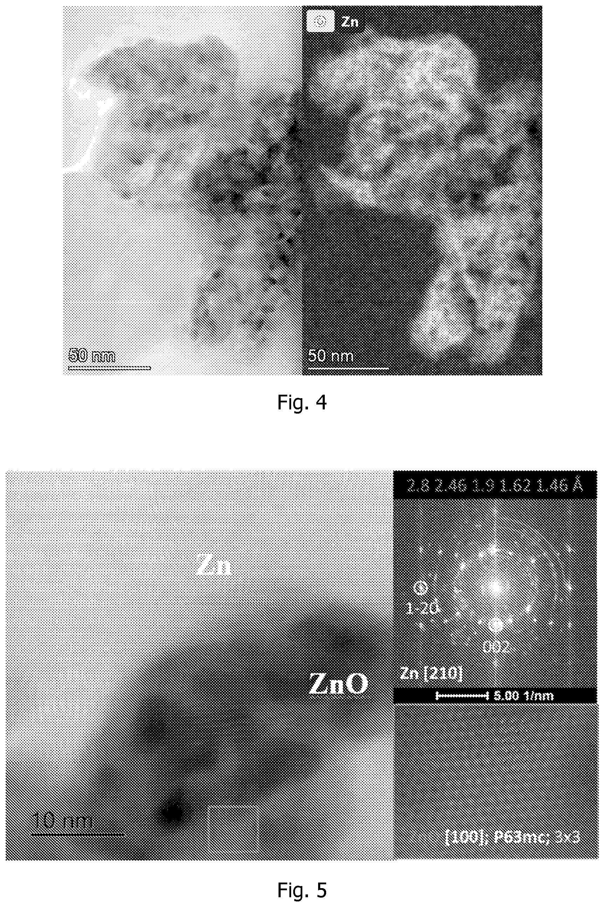

Shear introduced during hydro-extrusion deformed and fragmented ZnO passivation layers, which existed on an atomized Zn powder. Fragmented ZnO platelets i.e., very fine dispersoids, mostly resided at HAGB (**Fig. 3a, 3b**). The presence of crystalline ZnO dispersoids was approved by x-ray diffraction. As determined by hot gas fusion analysis the content of ZnO dispersoids in Zn matrix was rather small reaching 2.13 vol.%. The size of ZnO dispersoids, forming a porous, spongy-like structure was in general ~100 nm (**Fig. 4,5**). From a microscopic point of view, the ZnO dispersoids were evenly dispersed in Zn matrix, while the estimated interparticle spacing was a couple of hundreds nm. The crystalline structure of the platelet agglomerates corresponded to the XRD results. HE Zn+ZnO showed fully recrystallized and fine Zn grains decorated with ZnO nanoscale dispersoids. An average transversal grain size of HE Zn+ZnO, determined by EBSD, was 0.75 µm. It pointed to an effective stabilization of refined Zn grain structure by introduced ZnO dispersoids against recrystallization and grain growth during and after HE process.

### Mechanical testing

**Table 2. Ultimate tensile strength (UTS), 0.2% strain offset yield stress (YS_{0.2}), ductility (ε), and E of Zn+ZnO in as-extruded state fabricated by HE (HE Zn+ZnO) and after annealing at 100 °C for 24 h (HE+A Zn+ZnO). For comparison data on Zn+ZnO in as-extruded state fabricated by conventional extrusion (CE Zn+ZnO), cast Zn, Zn in as-extruded state (HE Zn) and after annealing at 100 °C for 24 h (HE+A Zn) is included.**

| **condition** | **UTS, MPa** | **YS_{0.2}, MPa** | **ε, %** | **E, GPa** |
|---|---|---|---|---|
| **HE Zn+ZnO** | 153 | 129 | 41 | 89.6 |
| **HE+A Zn+ZnO** | 153 | 143 | 39 | - |
| **CE Zn+ZnO** | 122 | 94 | 70 | - |
| **cast Zn** | 8 | 7 | 3 | 99 |
| **HE Zn** | 139 | 70 | 44 | 85.7 |
| **HE+A Zn** | 130 | 59 | 25 | - |

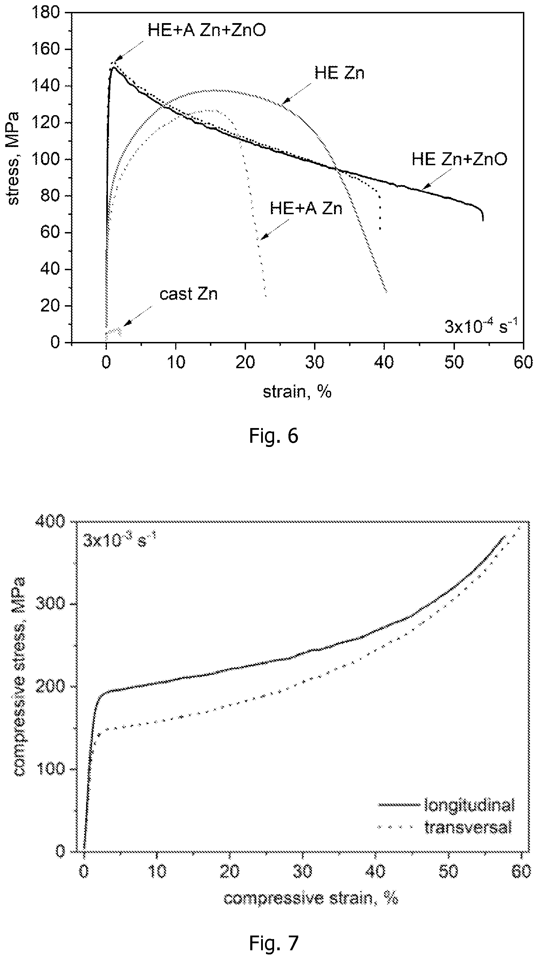

Application of HE was proven to enhance significantly the mechanical properties of the cast Zn. It was explained by an efficient grain refinement induced during low-temperature straining and partial suppression of DRX. In the present invention we confirmed that the poor mechanical properties, strengths, as well as ε of cast Zn improved noticeably, more than by an order of magnitude, after the application of HE deformation **(****Fig. 6****, Tab. 2).** HE accommodated substantial grain refinement and the area of GB increased markedly. GB worked as efficient obstacles, which impeded dislocation slip and promoted GB sliding, allowing for strengthening and greater plasticity at the same time. The mechanical properties of HE Zn+ZnO manufactured under the same conditions as HE Zn were superior to those of the HE Zn counterpart. The stress-strain curve of HE Zn+ZnO differed markedly from the one seen for HE Zn (**Fig. 6**).

While UTS of HE Zn+ZnO increased slightly by 10%, YS_{0.2} was boosted by 84% compared to the values determined for HE Zn counterpart. This could be rationalized by a positive effect of fine ZnO dispersoids. On one hand, ZnO positioned dominantly at HAGB didn't contribute to the Orowan strengthening mechanism markedly and they didn't act directly as obstacles to moving dislocations. On the other hand, ZnO dispersoids accommodated intensive grain refinement of Zn grain structure due to particle-induced nucleation and Zener pinning action, which resulted in an increase of GB mediated strengthening. In addition, ZnO presence at HAGB impeded mechanically GB sliding deformation mechanism, allowing for higher YS0.2. An excellent trade-off between strengths and ε of HE Zn+ZnO can be attributed to the bimodal Zn grain size distribution too. Owing to a minor texture and the presence of some elongated Zn grains higher strength of HE Zn+ZnO was confirmed in the L rather than in the T direction **(****Fig. 7**). The efficiency of the HE deformation process realized at the hydrostatic and almost frictionless conditions was demonstrated for Zn+ZnO extruded at the same parameters but using CE set-up. First, HE yielded a significantly reduced breakthrough pressure of 890 MPa compared to the extremely high value of 1500 MPa determined during CE, which was at the limit of utilized laboratory CE set-up with high-pressure capacity. Second, in comparison to CE Zn+ZnO the UTS and YS_{0.2} values of HE Zn+ZnO increased by ~25 and 37%, respectively.

On one hand, DRX accompanied by microstructure coarsening characteristically takes place in low-alloyed Zn. On the other hand, natural ageing processes active during storage and service in precipitation strengthened Zn alloys e.g., typically by Zn₁₁Mg₂ precipitates, lead to instability of the mechanical properties. In the absence of externally applied stress, a concept of stabilization by stable and fine dispersoids evenly distributed in the metallic matrix is effective up to high homologous temperatures of 0.93-0.99. An actual stabilization effect induced by ZnO dispersoids, which pinned Zn grain structure, was further demonstrated for HE Zn+ZnO annealed at 100 °C for 24 h. The testing temperature of 100 °C represented a homologous temperature of 0.54. The annealing at 100 °C for 24 h may be considered as an accelerated thermal treatment, which simulates long-term application of biomedical device at THB. Annealing didn't lead to microstructural change and grain growth, which would be accompanied by a strength decline inevitably. Conversely, the strengths, as well as ε improved slightly further for HE+A Zn+ZnO. Compared to HE Zn+ZnO YS_{0.2} increased by 11 % after annealing. This behaviour contradicted with ZnO free HE+A Zn subjected to the same thermal treatment, where an obvious deterioration of the mechanical properties took place. Compared to as-extruded conditions UTS, YS_{0.2} and ε of annealed HE+A Zn decreased by 7, 18 and 76%, respectively. It's worth pointing out, that to our knowledge HE+A Zn+ZnO material in the present invention showed the highest UTS, YS_{0.2} as well as ε ever reported for pure Zn.

HE Zn+ZnO reached E of 89.6 GPa, what was significantly less than the value of the cast Zn (99 ± 1.9 GPa), **Tab. 2.** The difference was attributed to a microstructural anisotropy, namely a pronounced texture in HE Zn+ZnO. HE Zn showed E of 85.7 GPa, which was ~5% less compared to that of the ZnO dispersoids containing counterpart. The increased E of HE Zn+ZnO compared to the one of HE Zn can likely be attributed to the presence of ZnO dispersoids, with a higher E of 140 GPa than that of the pure Zn. Also, the difference in the materials texture could contribute to the increase in E of the HE Zn+ZnO. It is worth pointing out that E plays a crucial role in the functioning of OIF and ES. On one hand, E ought to be as high as possible to avoid ES recoil after balloon deflation and to meet allowable elastic recoil on the expansion (<4%), eventually. An alternative criterion of expected recoil upon deflation of the balloon is defined by YS_{0.2} to E ratio and shall be in the range of 0.16-0.32% [10], while for HE+A Zn+ZnO this value (0.17%) fulfils this criterion. On the other hand, low E minimizes the stress-shielding phenomenon and thus improves a load transfer from OIF to the bone during its healing period service. Therefore, E=89.6 GPa of HE Zn+ZnO favours slightly better OIF rather than ES application.

### Stabilization

By ZnO dispersoids pinning Zener pressure was exerted on high angle grain boundaries (HAGB) and the driving force for migrating grain boundaries (GB) was balanced by the pinning action of ZnO dispersoids. Herein, stable nano-scaled ZnO dispersoids with the average size of 136 nm and 4.75 vol.% content, which were homogenously distributed in Zn matrix, provided stabilization of a grain structure down with the grain size of 0.75 µm. The actual stabilization effect induced by ZnO dispersoids was further demonstrated for Zn+ZnO annealed at 100 °C for 24 h. The testing temperature of 100 °C represented a homologous temperature (HT) of 0.54. Thus, the annealing at 100 °C for 24 h may be considered as an accelerated thermal treatment, which simulates a long-term application of biomedical device at THB. EBSD analyses confirmed that annealing didn't lead to major microstructural changes when compared to as-extruded Zn+ZnO. All these findings pointed to an effective stabilization of the refined Zn grain structure by introduced ZnO dispersoids that inhibited grain growth during and after the hydro-extrusion (HE) process. In other words, this mechanism enabled fabrication of ultrafine-grained Zn structure, which can be preserved during subsequent additional deformation, storage, and service up to 100 °C (**Fig. 6**).

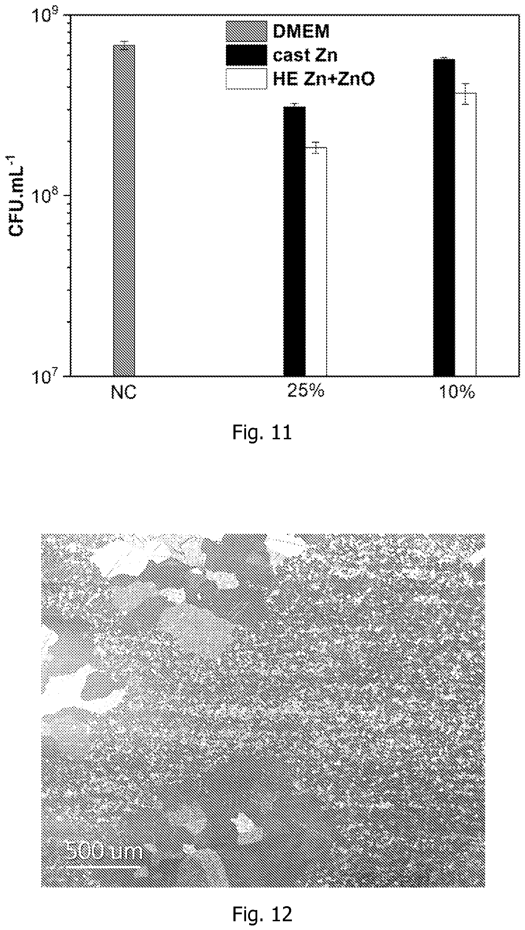

Stabilizing effect of UFG Zn structure by induced ZnO dispersoids was demonstrated through comparison with the non-stabilized fine-grained (18.4 µm) microstructure of HE Zn. HE Zn exposed to the annealing at 100 °C for 24 h underwent rather massive changes due to the grain growth (**Fig. 12**). A bimodal grain structure of HE+A Zn was confirmed, where approximately 60% of the volume of the grains remained nearly unaffected (22.2 µm). The rest of the grains experienced massive growth due to the recovery and recrystallization. The average diameter of the coarse equiaxed grains lied in the range of 50 to 210 µm, but exceptionally some grains had an average diameter higher than 1.5 mm. This was accompanied with inevitable deterioration of tensile properties of HE+A Zn (**Fig. 6**).

### Corrosion testing

The degradation behaviour of HE Zn+ZnO and cast Zn was accessed by static immersion and immersion tests. The apparent CR after 2 weeks of immersion in DMEM calculated for each group of materials was 0.021±0.003 and 0.015±0.001 mm·y⁻¹ for HE Zn+ZnO and cast Zn, respectively. The values agreed with those one reported in the literature for pure Zn and its alloys.

### In-vitro biological evaluation

The biocompatibility of the HE Zn+ZnO samples were evaluated via cytotoxicity, genotoxicity, oxidative stress, and bactericidal activity assays according to ISO 10993-5. The results were compared directly to cast Zn, of which *in-vitro* and *in-vivo* responses are well known and have been studied in details.

### Cytotoxicity assays

The indirect contact cytotoxicity of the studied samples was evaluated using a colorimetric MTT assay. According to the ISO 10993-5 criteria, cell viability values above 70% are considered a non-cytotoxic response. The test was done in pentaplicates and repeated three times to assure reproducible results. The potential cytotoxic responses of the extraction media of the HE Zn+ZnO and cast Zn materials are shown in **Fig. 11****.** In general, both materials exhibited rather comparable responses. The 100% extracts of both materials imposed a strong cytotoxic effect on the L929 cells after 24 and 72 h treatments. Conversely, 25 and 10% extracts resulted in non-cytotoxic response i.e., the cell viability value was more than 70%, which is considered as a non-cytotoxic behavior according to the ISO 10993-5 criteria. A relatively high cell viability can be seen for 25% extracts that were incubated for 72 h compared to those treated for 24 h.

### Genotoxicity

The genotoxic potential of the studied samples was determined by an alkaline comet assay (single-cell gel electrophoresis, SCGE). This method was performed to detect DNA damage in the incubated cells. The percentage of DNA in the tail (% tail DNA) was used as a parameter to detect DNA damage (DNA strand breaks). Six hundred comets were scored per sample in one electrophoresis run. In this analysis, the cells were treated with 500 µM H₂O₂ and serviced as a positive control (PC). As is shown in **Fig. 12****,** the incubation with the 100% extracts of both materials for 24 and 72 h resulted in a considerable percentage of DNA in the tail, and this behavior was comparable to that obtained for PC. The results showed that the incubation with the 25 and 10% extracts of both materials for 24 and 72 h resulted in no considerable DNA damage, and were very comparable to those obtained for NC. This behavior confirmed that DNA remained intact and no genotoxic responses were induced due to the treatment of diluted extracts. This behavior was consistent with the above-presented cytotoxic potential investigations.

### Oxidative stress testing

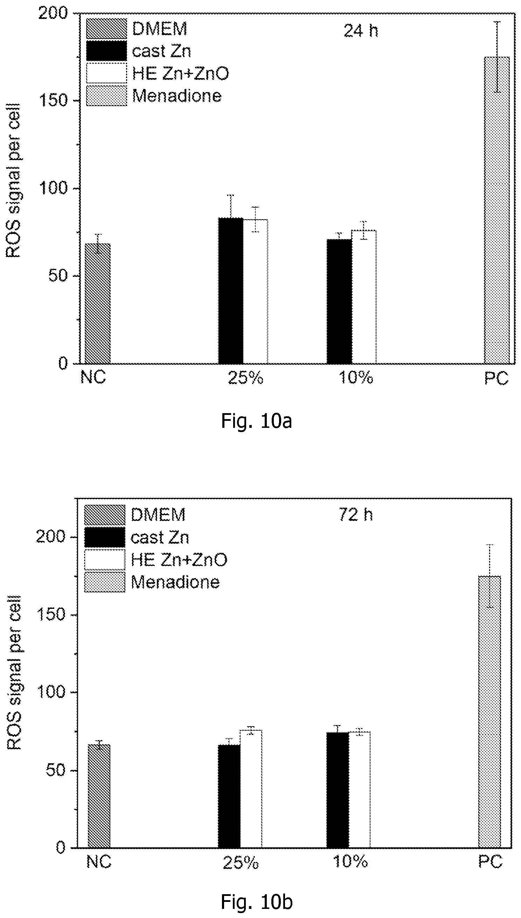

Formation of reactive oxygen species (ROS) was evaluated using a Molecular Probes CellROX Green Dye Oxidative Stress Reagent (ThermoFisher Scientific). An increase in the intracellular ROS level generally refers to a rise in the oxidative stress. **Fig. 10a, 10b** shows the ROS generation potential due to the exposure of the extracts of HE Zn+ZnO and cast Zn to L929 for 24 and 72 h. Following the above-presented results of the MTT and comet assays, only 25 and 10% extracts of both Zn materials were used for ROS as the execution of ROS for 100% extracts was pointless.

### Bactericidal activity

The antibacterial activity was assessed using Staphylococcus aureus (ATCC 6538). The bacterial suspension with a 10 µL volume containing ten colony forming units (CFU) was inoculated into 1 mL of the diluted (25 and 10%) extracts of HE Zn+ZnO and cast Zn specimens. Then it was incubated for 24 h at 37 °C. Subsequently, the bacterial suspension was diluted using the plating gradient dilution method from 10⁻¹ to 10^{-6,} and 100 µL of each dilution was inoculated onto a Petri dish with tryptone soya agar (TSA) plates in duplicates. After 24 h of growth at 37 °C, the bacterial colonies were counted and recalculated to CFU per 1 mL. As presented in **Fig. 11****,** fewer bacterial colonies grew in the diluted extracts of both materials after the incubation compared to control DMEM fresh, evidencing a small but still significant bacteriostatic activity of both materials.

The concertation of the Zn ions in the original extracts of cast Zn and HE Zn+ZnO was 330 and 280 µM·L⁻¹, respectively, whereas for the fresh DMEM medium it was 2.6 µM·L⁻¹. This represented 82.5 and 70 µM·L⁻¹ concentrations of Zn ions in 25% extracts of the cast Zn and HE Zn+ZnO, respectively. Thus, the Zn concentration of 82.5 µM represented the level that would not lead to any adverse effects, which was consistent with the previously reported results on the tolerance limits presented in [11]. It has been shown repeatedly that the cytotoxicity results (cell viability results) do not lead to the same conclusions as *in-vivo* tests [12]. It is generally considered that 5-8 times dilution of extracts during *in-vitro* testing can bring similar results to *in-vivo* tests*,* which was in an agreement with the findings in the present work. This was supported by successful *in-vivo* studies, which reported no adverse effects for the animal models despite apparent toxicity shown in vitro with non-diluted extracts [13].

Novel biomedical Zn+ZnO MMC was manufactured by PM approach using HE compaction of fine gas-atomized high purity Zn powder feedstock. The microstructure, mechanical properties, post-production stability, corrosion behaviour and in-vitro biological evaluation were pursued.

The results were compared with those obtained for the cast Zn reference. The following conclusions were drawn:
- HE resulted in the sound composite material characterized by a refined Zn matrix grain structure with the average grain size of 0.75 µm. Zn grain structure was formed dominantly by the fine recrystallized equiaxed grains and coarser fragmented grains elongated along the extrusion direction.
- 4.75 vol.% of ZnO dispersoids with the size of ~100 nm, which stemmed from passivating ZnO films on as-atomized Zn powder, were evenly dispersed within Zn grain structure and resided dominantly at Zn HAGB.
- ZnO dispersoids, which effectively refined Zn grain structure during HE, grain pinned Zn structure during post annealing realized at 100 °C for 24 h by Zener stabilization and thus prevented it from unwanted microstructural changes e.g., coarsening.
- Annealed Zn+ZnO showed the tensile mechanical properties, which were the values superior to all those reported for pure Zn in the literature.
- The *in-vitro* biological assays for the extracts (100, 25, and 10%) of the Zn+ZnO and cast Zn materials incubated with L929 resulted in comparable responses. 100% extracts imposed a strong toxic effect, while the 25 and 10% extracts resulted in a desirable response, which was rationalized on a basis of the highest safe Zn ion concentration.
- Small but still statistically significant bacteriostatic activity was confirmed for the extracts of HE Zn+ZnO incubated with Staphylococcus aureus, which was attributed to the presence of ZnO dispersoids.

### Example 3

**Table 3**

| **Sample #** | **Powder composition** | **Powder size d50 [µm]** | **Reduction ratio** | **% vol. of ZnO** | **UTS, MPa** | **YS_{0.2}, MPa** | **ε, %** |
|---|---|---|---|---|---|---|---|
| 1 | Zn/ZnO | 10.5 | 10:1 | 0.9 | 131 | 123 | 51 |
| 2 | Zn/ZnO | 2.1 | 15:1 | 5.1 | 155 | 130 | 40 |
| 3 | Zn/ZnO | 1.5 | 15:1 | 5.5 | 160 | 133 | 35 |
| 4 | Zn/ZnO | 0.9 | 20:1 | 8.0 | 167 | 138 | 30 |
| 5 | Zn/ZnO | 0.6 | 22:1 | 9.1 | 175 | 145 | 25 |

The composite materials shown in **Tab. 3** were prepared by the method described in Example 1. The composites were subjected to similar characterization and testing as in Example 1 with the following results:
- The strengths (YS_{0.2} and UTS) of the Zn+ZnO composites increased with the decrease of the grain size. The ductility of the Zn+ZnO composites decreased with the decrease of the grain size but this decrease was rather minor.
- Regardless of the amount of induced ZnO component, thermal stability of refined Zn grain structure attained by pinning action of Zn component was assured.
- The ductility of the samples 1 to 5 was sufficiently high for the application of ES and OIF application.

### Example 4

**Table 4**

| Sample # | **Powder composition** | **Powder size d50 [µm]** | **Reduction ratio** | % vol. of ZnO | **UTS, MPa** | **YS_{0.2}, MPa** | **ε, %** |
|---|---|---|---|---|---|---|---|
| 6 | Zn-0.8Mg/ZnO | 3.5 | 12:1 | 4.75 | 181 | 148 | 40 |
| 7 | Zn-0.8Mg/ZnO | 2.1 | 12:1 | 5.1 | 189 | 154 | 35 |
| 8 | Zn-1Ca/ZnO | 3.5 | 12:1 | 4.75 | 175 | 139 | 41 |
| 9 | Zn-1Ca-0.8Mg/ZnO | 2.1 | 12:1 | 5.1 | 181 | 142 | 36 |

The composite materials shown in **Tab. 4** were prepared by the method described in Example 1. The composites were subjected to similar characterization and testing as in Example 1 with the following results:
- Owing to fast-cooling rates present during gas atomization processed used to produce both fine nature Zn alloyed powders, Mg and Ca elements were found present mostly in a solid solution of Zn cell structure. Such structure was preserved during HE process realized at RT and no precipitation of e.g., Zn₁₁Mg₂ took place upon HE and long term storage at the temperature of a human body. At the same time ZnO component effectively stabilized the Zn grain structure and no grain growth took place.
- The strengths (YS_{0.2} and UTS) of the samples 6 to 9 increased with the decrease of the grain size.
- Significant increase of the strengths (YS_{0.2} and UTS) was confirmed compared to Zn+ZnO counterparts with the same Zn grain size.
- The ductility of the samples 6 to 9 was sufficiently high for the application of ES and OIF application.

### Example 5

**Table 5**

| Sample # | **Powder composition** | **Powder size d50 [µm]** | **Reduction ratio** | % vol. of the layer | **UTS, MPa** | **YS_{0.2}, MPa** | **ε, %** |
|---|---|---|---|---|---|---|---|
| 10 | Zn/MgO | 3.5 | 12:1 | 4.8 | 151 | 128 | 41 |
| 11 | Zn/ZnHPO₄ | 3.5 | 12:1 | 4.9 | 152 | 126 | 42 |

The composite materials shown in **Tab. 5** were prepared by the method described in Example 1. The composites were subjected to similar characterization and testing as in Example 1 with the following results:
- Fracture of MgO and ZnHPO₄ layers takes place during HE process to similar matter as the once determined for untreated Zn powder and native passivating ZnO films.
- MgO and ZnHPO₄ dispersoids provided similar stabilization effect to Zn grain structure as for untreated Zn powder and native passivating ZnO films and hence similar mechanical properties were obtained.

### References

[1] J.A. Finegold, P. Asaria, D.P. Francis, Int. J. Cardiol. 168 (2013) 934e945.
[2] S. Cook, P. Wenaweser, M. Togni, M. Billinger, C. Morger, C. Seiler, R. Vogel, O. Hess, B. Meier, S. Windecker, Circulation 115 (2007) 2426e2434.
[3] J. Venezuela, M.S. Dargusch, Acta Biomater. 87 (2019) 1.
[4] C.J. Frederickson, J.Y. Koh, A.I. Bush, Nat. Rev. Neurosci. 6 (2005) 449.
[5] P.K. Bowen, J. Drelich, J. Goldman, Adv. Mater. 25 (2013) 2577.
[6] H. Jin, S. Zhao, R. Guillory, P.K. Bowen, Z.Y. Yin, A. Griebel, J. Schaffer, E.J. Earley, J. Goldman, J.W. Drelich, Mater. Sci. Eng. C 84 (2018) 67.
[7] H. Yang, B. Jia, Z. Zhang, X. Qu, G. Li, W. Lin, D. Zhu, K. Dai, Y. Zheng, Nat. Commun. 11:401 (2020) 1-16.
[8] K. Piela, L. Blaz, W. Bochniak, P. Ostachowski, M. Lagoda, P. Zabinski, M. Jaskowski, M. Kiper, A. Polkowska, J. Alloys Compd. 810 (2019) 151883.
[9] E. Mostaed, M. Sikora-Jasinska, M.S. Ardakani, A. Mostaed, I.M. Reaney, J. Goldman, J.W. Drelich, Acta Biomater. 105 (2020) 319.
[10] P. Poncin, J. Proft, Sten Tubing: Understanding of the Desired Attributes, Medical Devices Materials: Proceedings of the Materials & Processes for Medical Devices Conference (2004) 253.
[11] J. Cheng, B. Liu, Y.H. Wu, Y.F. Zheng, J. Mater. Sci. Technol. 29 (2013) 619.
[12] I.V. Okulov, U. Kühn, J. Romberg, I.V. Soldatov, J. Freudenberger, L. Schultz, A. Eschke, C.-G. Oertel, W. Skrotzki, J. Eckert, Mater. Des. 62 (2014) 14.
[13] H. Yang, C. Wang, C. Liu, H. Chen, Y. Wu, J. Han, Z. Jia, W. Lin, D. Zhang, W. Li, W. Yuan, H. Guo, H. Li, G. Yang, D. Kong, D. Zhu, K. Takashima, L. Ruan, J. Nie, X. Li, Y. Zheng, Biomaterials 145 (2017) 92.

## Claims

1. A biocompatible and bioabsorbable metal matrix composite for manufacturing biomedical elements for full absorption *in vivo* in contact with a human or animal tissue obtainable by a method where a powder:
- having a mean particle size in a range from 50 to 10000 nm, where the powder particles consist of a core and an outer layer, and where the core is selected from a group consisting of: Zn, ZnMg alloy, ZnCa alloy; and the outer layer is selected from a group consisting of: ZnO, MgO, ZnHPO₄; and where the outer layer constitutes 0.1 to 10 vol. % of the powder particles;
is compacted by a cold isostatic pressing at a pressure from 20 to 400 MPa and subsequently extruded at a temperature below melting point of the powder at a reduction ratio 5:1 to 100:1, wherein the mean particle size of the powder and the reduction ratio are such as to obtain an ultra-fine grained matrix structure of the processed core material.

2. A biocompatible and bioabsorbable metal matrix composite according to claim 1, wherein the powder is extruded at the temperature in the range from 4 °C to melting point of the powder, preferably from 4 °C to 100 °C, the most preferably at a room temperature.

3. A biocompatible and bioabsorbable metal matrix composite according to claim 1 or 2, wherein the powder is cold compacted by a cold isostatic pressing at a pressure 100 to 300 MPa, preferably at 200 MPa.

4. A biocompatible and bioabsorbable metal matrix composite according to any of claim 1 to 3, wherein the powder has a mean particle size in the range from 500 to 10000 nm, preferably 1000 to 5000 nm, the most preferably 3000 nm.

5. A biocompatible and bioabsorbable metal matrix composite according to any of claim 1 to 4, where the outer layer constitutes 0.1 to 8 vol. %, preferably 1 to 7 vol. % of the powder particles, the most preferably 3 to 5 vol. % of the powder particles.

6. A biocompatible and bioabsorbable metal matrix composite according to any of claim 1 to 5, wherein the powder is extruded at the reduction ratio 10: 1 to 20:1, preferably 12:1.

7. A biocompatible and bioabsorbable metal matrix composite according to any of claim 1 to 6, wherein the extrusion is hydro-extrusion.

8. Endovascular stent or an orthopaedic internal fixator consisting of the metal matrix composite according to any of the preceding claims.

9. A method of manufacturing of a biocompatible and bioabsorbable metal matrix composite according to any of the claims 1 to 7, suitable for biomedical elements for full absorption *in vivo* in contact with a human or animal tissue, wherein a powder:
- having a mean particle size in the range from 50 to 10000 nm, where powder particles consist of a core and an outer layer, and where the core is selected from the group consisting of: Zn, ZnMg alloy, ZnCa alloy; and the outer layer is selected from the group consisting of: ZnO, MgO, ZnHPO₄; and where the outer layer constitutes 0.1 to 10 vol. % of the powder particles;
is compacted by cold isostatic pressing at a pressure from 20 to 400 MPa and subsequently extruded at the temperature below melting point of the powder at the reduction ratio 5:1 to 100:1, wherein the mean particle size of the powder and the reduction ratio are such as to obtain an ultra-fine grain matrix structure of the processed core material.

10. The method according to claim 9, wherein the powder is extruded at the temperature in the range from 4 °C to melting point of the powder, preferably from 4 °C to 100 °C, the most preferably at a room temperature.

11. The method according to any of claims 9 or 10, wherein the powder is cold compacted by a cold isostatic pressing at a pressure 100 to 300 MPa, preferably at 200 MPa.

12. The method according to any of claims 9 to 11, wherein the powder has a mean particle size in the range from 500 to 10000 nm, preferably 1000 to 5000 nm, the most preferably 3000 nm.

13. The method according to any of claims 9 to 12, where the outer layer constitutes 0.1 to 8 vol. %, preferably 1 to 7 vol. % of the powder particles, the most preferably 3 to 5 vol. % of the powder particles.

14. The method according to any of claims 9 to 13, wherein the powder is extruded at the reduction ratio 10:1 to 20:1, preferably 12:1.

15. The method according to any of claims 9 to 14, wherein the extrusion is hydro-extrusion.

## Patentansprüche

1. Ein biokompatibles und bioresorbierbares Metall-Matrix-Komposit zur Herstellung biomedizinischer Elemente zur vollständigen Resorption *in vivo* bei Kontakt mit menschlichem oder tierischem Gewebe, erhältlich durch ein Verfahren, bei dem ein Pulver:
- eine mittlere Partikelgröße im Bereich von 50 bis 10000 nm aufweist, wobei die Pulverpartikel aus einem Kern und einer Außenschicht bestehen und wobei der Kern aus einer Gruppe ausgewählt ist, die aus Zn, ZnMg-Legierung, ZnCa-Legierung besteht; und die Außenschicht aus einer Gruppe ausgewählt ist, die aus ZnO, MgO, ZnHPO₄ besteht; und wobei die Außenschicht 0,1 bis 10 Vol.-% der Pulverpartikel ausmacht;
durch kaltisostatisches Pressen bei einem Druck von 20 bis 400 MPa verdichtet und anschließend bei einer Temperatur unterhalb des Schmelzpunkts des Pulvers bei einem Reduktionsverhältnis von 5:1 bis 100:1 extrudiert wird, wobei die mittlere Partikelgröße des Pulvers und das Reduktionsverhältnis so beschaffen sind, dass eine ultrafeinkörnige Matrixstruktur des verarbeiteten Kernmaterials erhalten wird.

2. Ein biokompatibles und bioresorbierbares Metall-Matrix-Komposit nach Anspruch 1, wobei das Pulver bei einer Temperatur im Bereich von 4 °C bis zum Schmelzpunkt des Pulvers, vorzugsweise von 4 °C bis 100 °C, am bevorzugtesten bei Raumtemperatur extrudiert wird.

3. Ein biokompatibles und bioresorbierbares Metall-Matrix-Komposit nach Anspruch 1 oder 2, wobei das Pulver durch kaltisostatisches Pressen bei einem Druck von 100 bis 300 MPa, vorzugsweise bei 200 MPa, kalt verdichtet wird.

4. Ein biokompatibles und bioresorbierbares Metall-Matrix-Komposit nach einem der Ansprüche 1 bis 3, wobei das Pulver eine mittlere Partikelgröße im Bereich von 500 bis 10000 nm, vorzugsweise 1000 bis 5000 nm, am bevorzugtesten 3000 nm aufweist.

5. Ein biokompatibles und bioresorbierbares Metall-Matrix-Komposit nach einem der Ansprüche 1 bis 4, wobei die Außenschicht 0,1 bis 8 Vol.-%, vorzugsweise 1 bis 7 Vol.-% der Pulverpartikel, am bevorzugtesten 3 bis 5 Vol.-% der Pulverpartikel ausmacht.

6. Ein biokompatibles und bioresorbierbares Metall-Matrix-Komposit nach einem der Ansprüche 1 bis 5, wobei das Pulver mit einem Reduktionsverhältnis von 10:1 bis 20:1, vorzugsweise 12:1, extrudiert wird.

7. Ein biokompatibles und bioresorbierbares Metall-Matrix-Komposit nach einem der Ansprüche 1 bis 6, wobei die Extrusion eine Hydroextrusion ist.

8. Endovaskulärer Stent oder orthopädischer interner Fixateur, bestehend aus dem Metall-Matrix-Komposit nach einem der vorhergehenden Ansprüche.

9. Verfahren zur Herstellung eines biokompatiblen und bioresorbierbaren Metall-Matrix-Komposits nach einem der Ansprüche 1 bis 7, das für biomedizinische Elemente zur vollständigen Resorption *in vivo* bei Kontakt mit menschlichem oder tierischem Gewebe geeignet ist, wobei ein Pulver:
- eine mittlere Partikelgröße im Bereich von 50 bis 10000 nm aufweist, wobei die Pulverpartikel aus einem Kern und einer Außenschicht bestehen und wobei der Kern aus einer Gruppe ausgewählt ist, die aus Zn, ZnMg-Legierung, ZnCa-Legierung besteht; und die Außenschicht aus einer Gruppe ausgewählt ist, die aus ZnO, MgO, ZnHPO₄ besteht; und wobei die Außenschicht 0,1 bis 10 Vol.-% der Pulverpartikel ausmacht;
durch kaltisostatisches Pressen bei einem Druck von 20 bis 400 MPa verdichtet und anschließend bei einer Temperatur unterhalb des Schmelzpunkts des Pulvers bei einem Reduktionsverhältnis von 5:1 bis 100:1 extrudiert wird, wobei die mittlere Partikelgröße des Pulvers und das Reduktionsverhältnis so beschaffen sind, dass eine ultrafeinkörnige Matrixstruktur des verarbeiteten Kernmaterials erhalten wird.

10. Verfahren nach Anspruch 9, wobei das Pulver bei einer Temperatur im Bereich von 4 °C bis zum Schmelzpunkt des Pulvers, vorzugsweise von 4 °C bis 100 °C, am bevorzugtesten bei Raumtemperatur extrudiert wird.

11. Verfahren nach einem der Ansprüche 9 oder 10, wobei das Pulver durch kaltisostatisches Pressen bei einem Druck von 100 bis 300 MPa, vorzugsweise bei 200 MPa, kalt verdichtet wird.

12. Verfahren nach einem der Ansprüche 9 bis 11, wobei das Pulver eine mittlere Partikelgröße im Bereich von 500 bis 10000 nm, vorzugsweise 1000 bis 5000 nm, am bevorzugtesten 3000 nm aufweist.

13. Verfahren nach einem der Ansprüche 9 bis 12, wobei die Außenschicht 0,1 bis 8 Vol.-%, vorzugsweise 1 bis 7 Vol.-% der Pulverpartikel, am bevorzugtesten 3 bis 5 Vol.-% der Pulverpartikel ausmacht.

14. Verfahren nach einem der Ansprüche 9 bis 13, wobei das Pulver mit einem Reduktionsverhältnis von 10:1 bis 20:1, vorzugsweise 12:1, extrudiert wird.

15. Verfahren nach einem der Ansprüche 9 bis 14, wobei die Extrusion eine Hydroextrusion ist.

## Revendications

1. Composite à matrice métallique biocompatible et bioabsorbable pour la production d'éléments biomédicaux destinés à être entièrement absorbés *in vivo* au contact avec un tissu humain ou animal obtenable par un procédé dans lequelle une poudre:
- ayant une taille moyenne de particules comprise entre 50 et 10 000 nm, où les particules de poudre sont constituées d'un noyau et d'une couche externe, et le noyau est issu d'un groupe se composant de: Zn, alliage ZnMg, alliage ZnCa; et la couche externe est issue d'un groupe se composant de: ZnO, MgO, ZnHPO₄; et la couche externe constitue de 0,1 à 10 vol. % des particules de poudre;
est compactée par un pressage isostatique à froid à une pression de 20 à 400 MPa et successivement extrudée à une température inférieure au point de fusion de la poudre dans un rapport de réduction de 5:1 à 100:1, où la taille moyenne des particules de poudre et le taux de réduction permettent d'obtenir une structure matricielle à grains ultra fins du matériau de base traité.

2. Composite à matrice métallique biocompatible et bioabsorbable selon la revendication 1, où la poudre est extrudée à une température comprise dans un intervalle entre 4 °C et le point de fusion de la poudre, de préférence entre 4 °C et 100 °C, le plus préférentiellement à température ambiante.

3. Composite à matrice métallique biocompatible et bioabsorbable selon la revendication 1 ou 2, où la poudre est compactée par un pressage isostatique à froid à une pression de 100 à 300 MPa, de préférence 200 Mpa.

4. Composite à matrice métallique biocompatible et bioabsorbable selon l'une des revendications de 1 à 3, où la poudre a une taille moyenne comprise dans un intervalle de 500 à 10 000 nm, de préférence de 1000 à 5 000 nm, le plus préférentiellement 3 000 nm.

5. Composite à matrice métallique biocompatible et bioabsorbable selon l'une des revendications de 1 à 4, où la couche externe constitue de 0,1 à 8 vol. % des particules de poudre, de préférence de 1 à 7 vol. %, le plus préférentiellement de 3 à 5 vol. %.

6. Composite à matrice métallique biocompatible et bioabsorbable selon l'une des revendications de 1 à 5, où la poudre est extrudé à un taux de réduction de 10:1 à 20:1, de préférence 12:1.

7. Composite à matrice métallique biocompatible et bioabsorbable selon l'une des revendications de 1 à 6, où l'extrusion est une hydro-extrusion.

8. Stent endovasculaire ou fixateur interne orthopédique constitué du composite à matrice métallique selon l'une des revendications précédentes.

9. Un procédé de fabrication d'un composite à matrice métallique biocompatible et bioabsorbable selon l'une des revendications de 1 à 7, adapté aux éléments biomédicaux, pour être entièrement absorbé in vivo au contact avec un tissu humain ou animal, où une poudre:
- ayant une taille moyenne de particules comprise entre 50 et 10 000 nm, où les particules de poudre sont constituées d'un noyau et d'une couche externe, et le noyau est issu d'un groupe se composant de: Zn, alliage ZnMg, alliage ZnCa; et la couche externe est issue du groupe se composant de: ZnO, MgO, ZnHPO₄; et la couche externe constitue de 0,1 à 10 vol. % des particules de poudre;
est compactée par un pressage isostatique à froid à une pression de 20 à 400 MPa et successivement extrudée à une température inférieure au point de fusion de la poudre dans le rapport de réduction de 5:1 à 100:1, où la taille moyenne des particules de poudre et le taux de réduction permettent d'obtenir une structure matricielle à grains ultra fins du matériau de base traité.

10. Le procédé selon la revendication 9, où la poudre est extrudée à une température comprise dans un intervalle entre 4 °C et le point de fusion de la poudre, de préférence entre 4 °C et 100 °C, le plus préférentiellement à température ambiante.

11. Procédé selon l'une des revendications 9 ou 10, où la poudre est compactée par un pressage isostatique à froid à une pression de 100 à 300 MPa, de préférence 200 Mpa.

12. Procédé selon l'une des revendications de 9 à 11, où la poudre a une taille moyenne comprise dans un intervalle de 500 à 10 000 nm, de préférence de 1000 à 5 000 nm, le plus préférentiellement 3 000 nm.

13. Procédé selon l'une des revendications de 9 à 12, où la couche externe constitue de 0,1 à 8 vol. % des particules de poudre, de préférence de 1 à 7 vol. %, le plus préférentiellement de 3 à 5 vol. %.

14. Procédé selon l'une des revendications de 9 à 13, où la poudre est extrudé à un taux de réduction de 10:1 to 20:1, de préférence 12:1.

15. Procédé selon l'une des revendications 9 à 14, où l'extrusion est une hydro-extrusion.
